Europäisches Patentamt

European Patent Office

Office européen des brevets

(19)

(11) Publication number: **0 400 239**
A1

# (12) EUROPEAN PATENT APPLICATION

(21) Application number: 89307608.3

(22) Date of filing: 27.07.89

(51) Int. Cl.5: **C12P 7/02, C12P 7/26, C12P 7/62, C12P 11/00**

(30) Priority: 26.05.89 JP 133950/89

(43) Date of publication of application:
05.12.90 Bulletin 90/49

(84) Designated Contracting States:
CH DE LI

(71) Applicant: **DAITO KOEKI KABUSHIKI KAISHA**
**326, Yohkamachi**
**Toyama-shi Toyama-ken(JP)**

(72) Inventor: **Kometani, Tadashi**
**4, Daimon Daimon-machi**
**Imizu-gun Toyama-ken(JP)**
Inventor: **Fujimori, Yukio c/o Daito Koeki**
**Kabushiki Kaisha**
**326, Yohkamachi**
**Toyama-shi Toyama-ken(JP)**

(74) Representative: **Wilkinson, Stephen John et al**
**c/o Stevens, Hewlett & Perkins 5 Quality**
**Court Chancery Lane**
**London WC2A 1HZ(GB)**

(54) **Process for asymmetric reduction of ketones with baker's yeast.**

(57) A process for asymmetric reduction of ketones with baker's yeast, wherein ketones as a substrate are reacted in an aqueous solution by using ethanol as an energy source for the reduction under aerobic conditions.

Since evolution of gaseous carbon dioxide and formation of by-products can be suppressed and the concentration of the substrate can be increased, it is extremely useful for mass synthesis and practical application.

EP 0 400 239 A1

# PROCESS FOR ASYMMETRIC REDUCTION OF KETONES WITH BAKER'S YEAST

## BACKGROUND OF THE INVENTION

### Field of the Invention

The present invention concerns a process, with industrial advantage, for asymmetric reduction of ketones with baker's yeast.

### Description of the Prior Art

Asymmetric reduction with baker's yeast has been utilized in organic synthesis from about the year of 1980, and about one hundred kinds of substrates have been known therefor. Among all, asymmetric reduction of ketones with baker's yeast has been employed generally as a simple production process for chiral building blocks. As an example of such asymmetric reduction, there has been known a reaction of reducing ethyl ascetoacetate to obtain (S)-(+)-ethyl 3-hydroxybutanoate (Org. Synth. 63, 1 (1984)). This reaction is usually conducted in an aqueous 5 - 10 % solution of sugar (glucose, sucrose, etc.).

As has been described above, sugar is used as an energy source for the reduction in the prior art process. Accordingly, there has been a problem that a great amount of gaseous carbon dioxide and various kind of malodor-releasing by-products are formed in the course of the glucose metabolism. In addition, since a great amount of gaseous carbon dioxide evolved form gas bubbles, there has been attempted to add a defoamer comprising a surface active agent or the like for eliminating the bubbles. However, addition of the defoamer brings about a further problem of causing other reactions than the asymmetric reduction, failing to obtain an aimed product efficiently. Accordingly, the conventional process of using sucrose as the energy source for the reduction involves a problem to be solved, concerned with the mass synthesis and practical application.

## SUMMARY OF THE INVENTION

It is, accordingly, a first object of the present invention to overcome the foregoing problems in the prior art and provide a process for the asymmetric reduction of ketones with baker's yeast, enabling mass synthesis and practical application while suppressing the evolution of gaseous carbon dioxide and the formation of by-products.

A second object of the present invention is to provide a process for the asymmetric reduction of ketones with baker's yeast capable of obtaining an aimed product at a yield and an optical purity as comparable with or superior to those obtained by the conventional asymmetric reduction.

The present inventors has noted the foregoing problems in the asymmetric reduction of ketones with baker's yeast, have made various studies for the counter measures and, as a result, have accomplished the present invention based on the finding that when ketones, as a substrate, are brought into reaction under aerobic conditions by using ethanol as an energy source for the reduction, evolution of gaseous carbon dioxide and formation of by-products can be suppressed while proceeding the reduction quite in the same manner as in the conventional process.

That is, the present invention provides a process for the asymmetric reduction of ketones with baker's yeast in which ketones are brought into reaction under aerobic conditions by using ethanol as an energy source for the reduction.

## BRIEF DESCRIPTION OF THE DRAWING

These and other objects, as well as advantageous features of the present invention will become more apparent by reading the following description referring to a preferred embodiment in conjunction the accompanying drawings, wherein;

Fig. 1 is a graph illustrating the state of asymmetric reduction using glucose as an energy source;

Fig. 2 is a graph illustrating the state of asymmetric reduction using ethanol as an energy source; and

Fig. 3 is a graph showing the state of asymmetric reduction for comparison between ethanol and other alcohols used as the energy source.

## DETAILED DESCRIPTION OF THE INVENTION

The baker's yeast used in the present invention belongs to Saccharomyses cerevisiae having selectivity which is different depending on the substrate.

As the substrate, any of those having carbonyl groups with which reduction by baker's yeast proceeds by using sugar as an energy source may be used. Specifically, the process of the reduction according to the present invention can be applied with no particular restrictions to those reduction processes in which the substrate undergoes asymmetric reduction with baker's yeast in an aqueous solution of sugar typically represented by glucose and sucrose (Various examples of the reaction are collected in Journal of the Synthetic Organic Chemistry, Vol 44, No. 6, 519 (1986) and Vol 46, No. 8, 726 (1988)). As a typical example of such reduction, there can be mentioned reaction for obtaining (S)-(+)-ethyl 3-hydroxybutanoate from ethyl acetoacetate described above. Further as other examples, there can be mentioned reduction of ethyl 2-oxocyclohexane carboxylate into (+)-(1R, 2S) ethyl-cis-2-hydroxycyclohexane carboxylate (Helv. Chim. Acta, Vol. 63, 1383, (1980)), conversion of 1-chloro-3-phenyl sulfonyl-2-propanone into (2R)-1-chloro-3-phenyl sulfonyl-2-propanol (Synthesis, 389 (1987)), conversion of 2-acetoxy-1-phenyl ethanone into (S)-(+)-2-acetoxy-1-phenylethanol (J. Org. Chem. Vol. 53, 4405 (1988)).

The substrate usable in the asymmetric reduction of ketones is added into an aqueous solution of the reaction system at a concentration of not greater than 50 g/l and, desirably, at a concentration of not greater than 30 g/l. In particular, when the substrate is added to the aqueous solution of the reaction system at a concentration of from 15 g/l to 50 g/l, preferably, from 15 g/l to 30 g/l, the concentration of the substrate is increased and it is extremely effective, for example, for the mass synthesis in a reaction vessel. By the way, if the concentration of the substrate is increased in the conventional asymmetric reduction using sugar as the energy source, the addition amount of the sugar is also increased correspondingly and the evolution of gaseous carbon dioxide and the formation of the by-products are also increased making it difficult for the mass synthesis and practical application.

Ethanol used in the asymmetric reduction is added to the aqueous solution of the reduction system by from 0.1 to 10 vol%, desirably, from 0.5 to 5 vol%. If the addition amount of ethanol is not greater than 0.1 vol%, the energy source becomes insufficient to lower the reaction rate in the asymmetric reduction. On the other hand, if the addition amount of the ethanol is not less than 10 vol%, enzyme in the yeast is deactivated to lower the reaction rate remarkably.

The ratio of ethanol to the substrate is, preferably, from 1:1 to 1:10.

Then, in the asymmetric reduction according to the present invention, it is necessary to maintain the reaction system under aerobic conditions. For this purpose, it is possible to employ means for agitating an aqueous solution containing each of the ingredients described above and, at the same time, blowing oxygen or oxygen-containing gas (for example, air) into the aqueous solution, for example, in a reaction vessel where mass synthesis is intended.

In a case where the concentration of the substrate to the aqueous solution is increased, for example, to 15 -30 g/l under the state where the aerobic conditions are maintained, it is desirable that the amount of the substrate is divided into two - several portions and each of them is added together with ethanol into the aqueous solution of the reaction system at a predetermined time interval, so that the final concentration of the substrate is from 15 to 30 g/l.

Further, a buffer agent or the like may be added, as required, to the aqueous solution of the asymmetric reduction system for controlling the pH value of the reaction solution. Addition of the buffer can provide pH control in the reaction solution, thereby improving the yield of aimed products in the asymmetric reduction, etc.

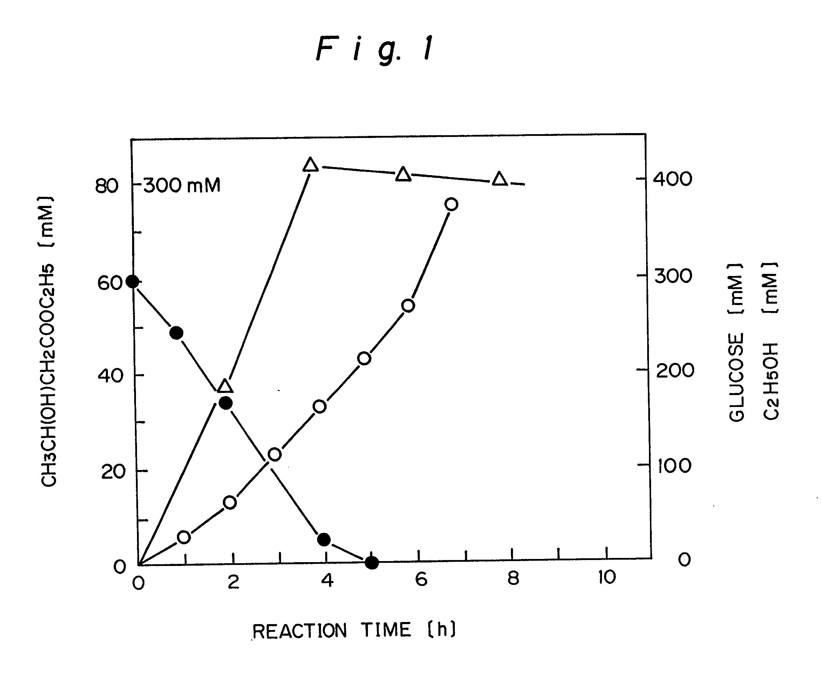

Fig. 1 shows a state when 300 mM of glucose, a substrate containing 77 mM of ethyl acetoacetate and baker's yeast were shaken at 30°C.

In Fig. 1, plotting shown by blank circles represent aimed product and plotting shown by solid circles represent glucose and plotting shown by blank triangle represent ethanol. As seen from Fig. 1, the reaction product ($CH_3CH(OH)CH_2COOC_2H_5$) increases with elapse of the reaction time, whereas glucose decreases with elapse of the reaction time. The yield of ethanol is rapidly increased and the product is increased even after the consumption of glucose.

From the result of Fig. 1, it has been estimated that ethanol formed in the glycolytic pathway of glucose much contributes to the asymmetric reduction.

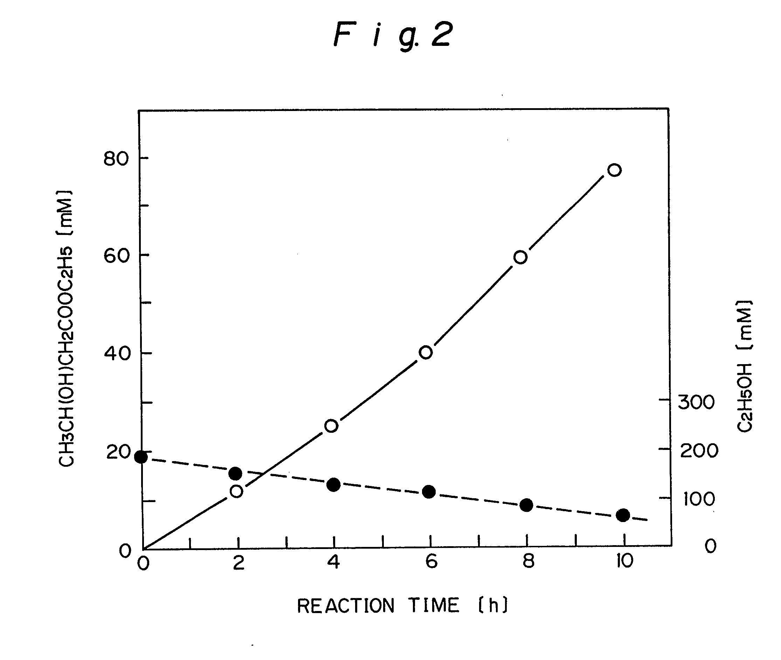

Fig. 2 shows the state in which 200 mM of ethanol and a substrate containing 77 mM of ethyl acetoacetate were shaken at 30°C.

In Fig. 2, plottings shown by blank circles represent product and plottings by solid circles represent ethanol.

As can be seen from Fig. 2, asymmetric reduction proceeds smoothly, ethanol is consumed along with elapse of the reaction time, in which the isolated yield for the product was from 60 to 70 % and the optical rotation was not less than +38.50 (ee 90%).

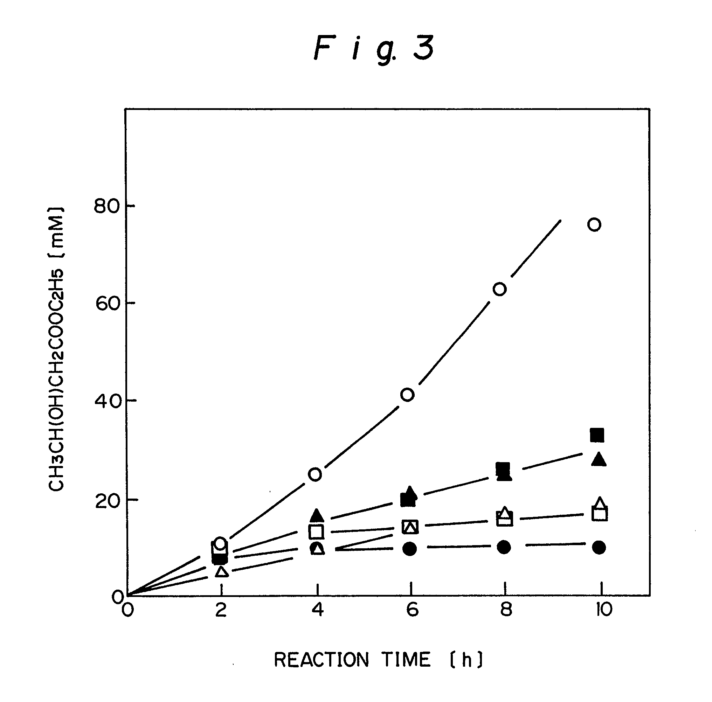

Then, Fig. 3 shows comparison between ethanol and other alcohols used as the energy source for the asymmetric reduction. Referring to the plottings shown in Fig. 3, blank circles represent ethanol, blank triangles represent methanol and blank squares represent normal propanol, solid circles represent normal butanol, solid trigonals represent ethylene glycol and solid squares represent glycerine. As can be seen from Fig. 3, the yield of the product is gradually increased along with the elapse of reaction time in the case of using ethanol as compared with other alcohols, showing that ethanol is more effective in the asymmetric reduction according to the present invention as compared with other alcohols.

## EXAMPLE

The present invention is to be described more specifically referring to examples.

## EXAMPLE 1

Baker's yeast (living yeast, manufactured by Oriental Yeast Co.) was suspended by 2.8 g into 50 ml of distilled water, to which a solution of 0.5 g of ethyl acetoacetate and 0.57 ml of ethanol was added and shaken at 30°C for 10 hours. After adding 2.8g of celite and stirring by a magnetic stirrer at a room temperature for one hour, they were filtered under suction and the filtrate was saturated with sodium chloride. Then, after extracting for three times each times with 25 ml of ethanol, the organic layer was dried with anhydrous magnesium sulfate and filtered. Then, the solvent was distilled off under a reduced pressure and the residue was subjected to distillation under a reduced pressure, to obtain 0.335 g (67%) of (S)-(+)-ethyl 3-hydroxybutanoxylate.
bp: 80 - 85°C (20 mmHg)
IR: 3450 cm$^{-1}$, 1730 cm$^{-1}$
NMR (CDCl$_3$):
1.18 (3H, d, J=6Hz), 1.24 (3H, t, J=7Hz), 2.35 (2H, d, J=8Hz), 3.58 (1H, s), 4.10 (2H, q, J=7Hz), 4.10 (1H, m)
$(\alpha)_D^{23}$ + 41.61° (C=1.00, CHCl$_3$)

## EXAMPLE 2

The same procedures as those in Example 1 were applied except for using 0.5 g of ethyl 2-oxocyclohexane carbonate instead of ethyl acetoacetate and shaking for 24 hours, to obtain 0.34 g (68%) of ethyl (+)-(1R, 2S)-2-hydroxycyclohexane carboxylate.
bp: 100 - 110°C (5 mmHg)
IR: 3450 cm$^{-1}$, 1740 cm$^{-1}$
NMR (CDCl$_3$):
1.28 (3H, t, J=7Hz), 1.20 - 2.00 (8H, m), 2.40 - 2.60 (1H, m), 3.10 (1H, br), 4.05 - 4.20 (1H, m), 4.18 (2H, q, J=8Hz)
$(\alpha)_D^{23}$ + 25.87° (C=1.23, CHCl$_3$)

## EXAMPLE 3

The same procedures as those in Example 1 were applied except for using 0.5 g of ethyl 2-oxocyclopentanecarboxylate instead of ethyl acetoacetate and shaking for 12 hours, to obtain 0.35 g (70%) of ethyl(+)-(1R, 2S)-2-hydroxycyclopentane carboxylate.
bp: 100 - 110°C (5 mmHg)
IR: 3450 cm$^{-1}$, 1730 cm$^{-1}$
NMR (CDCl$_3$):
1.20 (3H, t, J=7Hz), 1.40 - 2.05 (6H, m), 2.45 - 2.75 (1H, m), 2.60 - 3.00 (1H, br), 4.10 (2H, q, J=7Hz), 4.15 - 4.40 (1H, m)
$(\alpha)_D^{23}$ + 14.99° (C=1.20, CHCl$_3$)

## EXAMPLE 4

The same procedures as those in Example 1 were applied except for using 0.5 g of 1-phenyl-1,2-propanedione instead of ethyl acetoacetate and shaking for 12 hours, to obtain 0.38 g (76%) of (S)-(-)-2-hydroxy-1-phenyl-1-propanone.
bp: 130 - 140°C (20 mmHg)
IR: 3400 cm$^{-1}$, 1710 cm$^{-1}$
NMR (CDCl$_3$):
1.04 (3H, d, J=6.5Hz), 3.80 - 4.10 (1H, m), 4.62 (1H, d, J=4Hz), 7.31 (5H, s)
$(\alpha)_D^{23}$ - 39.40° (C=1.01, CHCl$_3$)

## EXAMPLE 5

The same procedures as those in Example 1 were applied except for using 0.5 g of 1-chloro-3-phenylsufonyl-2-propanone instead of ethyl acetoacetate and shaking for 24 hours, to obtain 0.375 g (75%) of (2R)-(+)-1-chloro-3-phenylsulfonyl-2-propanol.
mp: 87 - 89°C
IR: 3500 cm$^{-1}$
NMR (CDCl$_3$):
3.30 - 3.45 (3H, m), 3.55 - 3.70 (2H, m), 7.50 - 8.00 (5H, m)
$(\alpha)_D^{23}$ + 13.60° (C=1.00, CHCl$_3$)

## EXAMPLE 6

The same procedures as those in Example 1 were applied except for using 0.5 g of 2-acetoxy-1-phenylethanone instead of ethyl acetoacetate and shaking for 10 hours, to obtain 0.36 g (72%) of (S)-(+)-2-acetoxy-1-phenylethanol.
bp: 140 - 150°C (3 mmHg)
IR: 3450 cm$^{-1}$, 1730 cm$^{-1}$
NMR (CDCl$_3$):
1.97 (3H, s), 3.40 (1H, br), 4.05 - 4.25 (2H, m), 4.75 - 4.95 (1H, m), 7.30 (5H, s)

$( \alpha )_D^{23}$ + 56.03° (C = 1.01, CHCl₃)

EXAMPLE 7

Baker's yeast (living yeast, manufactured by Oriental Yeast Co.) was suspended by 2.8 g into 50 ml of distilled water, to which a solution of 0.375 g of ethyl acetoacetate and 0.43 mol of ethanol was added and shaken at 30°C for 8 hours. Further, 0.375 g of ethyl acetoacetate and 0.43 ml of ethanol were added and shaken at 30°C for 8 hours. After adding 2.8g of celite and stirring by a magnetic stirrer at a room temperature for one hour, they were filtered under suction and the filtrate was saturated with sodium chloride. Then, after extracting for three times each times with 25 ml of ethanol, the organic layer was dried with anhydrous magnesium sulfate and filtered. Then, the solvent was distilled off under a reduced pressure and the residue was subjected to distillation under a reduced pressure, to obtain 0.5 g (67%) of (S)-(+)-ethyl 3-hydroxybutanoxylate.

As a result of analysis for the product, it was confirmed that the data were quite identical with those in Example 1.

EXAMPLE 8

One liter of distilled water was added to 112 g of baker's yeast (living yeast, manufacture by Oriental Yeast Co.) and slowly stirred by a magnetic stirrer and, further, a solution of 10 g of ethyl acetoacetate and 11.5 ml of ethanol was added and reacted while blowing air into the liquid suspension by a small pump (at 1.5 l/min). After 24 hours, a solution of 10 g of ethyl acetoacetate and 11.5 ml of ethanol was again added and then reacted for 24 hours. A solution of 10 g of ethyl acetoacetate and 11.5 ml of ethanol was further added and then 120 g of celite was added and stirred by a magnetic stirrer at a room temperature for one hour. Subsequently, they were filtered under suction and the filtrate was saturated with sodium chloride. Then, after extracting for three times each time with 300 ml of ether, the organic layer was dried with anhydrous magnesium sulfate and filtered. The solvent was distilled off under a reduced pressure and the residue was subjected to distillation under a reduced pressure to obtain 18 g (60%) of (S)-(+)-ethyl-3-hydroxybutanoxylate.

As the result of analysis for the product, it was confirmed that the data were quite identical with those of Example 1.

EXAMPLE 9

Ethyl 2-oxocyclopentane carboxylate was used instead of ethyl acetoacetate, which was added portionwise for three times each time with 10 g, thus, 30 g in total to one litre of distilled water and then reacted for 72 hours in total in the same manner as in Example 3, to obtain 21 g (70%) of ethyl (+)-(1R, 2S)-2-hydroxycyclopentane carboxylate.

As a result of analysis for the obtained product, it was confirmed that the data were identical with those in Example 3.

EXAMPLE 10

2-acetoxy-1-phenyl ethanone was used instead of ethyl acetoacetate, which was added portionwise for three times each time with 10 g, thus, 30 g in total to one litre of distilled water and then reacted for 72 hours in total in the same manner as in Example 3, to obtain 21.5 g (72%) of (S)-(+)-2-acetoxy-1-phenylethanol.

As a result of analysis for the product, it was confirmed that the data were quite identical with those in Example 6.

According to the present invention, ethanol is used as the energy source for the asymmetric reduction of ketones. It is, therefore, possible to suppress the evolution of gaseous carbon dioxide and the formation of by-products in the conventional process using sugar as the energy source and increase the concentration of the substrate and, accordingly, it is extremely effective for the mass synthesis and practical application.

Claims

(1) A process for asymmetric production of ketones with baker's yeast, wherein the reaction is conducted in an aqueous solution containing ketones as a substrate under aerobic conditions by using ethanol as an energy source for the reduction.

(2) A process for asymmetric reduction of ketones with baker's yeast as defined in claim 1, wherein the substrate is added to the aqueous solution at a concentration of not greater than 50 g/l.

(3) A process for asymmetric reduction of ketones with baker's yeast as defined in claim 2, wherein the substrate is added to the aqueous solution at a concentration of not greater than 30 g/l.

(4) A process for asymmetric reduction of ketones with baker's yeast as defined in claim 1, wherein the ethanol is added by from 0.1 to 10 vol % to the aqueous solution.

(5) A process for asymmetric reduction of ketones with baker's yeast as defined in claim 4, wherein ethanol is added by from 0.5 to 5 vol % to the aqueous solution.

(6) A process for asymmetric reduction of ketones with baker's yeast as defined in claim 1, wherein the ratio of adding ethanol to the substrate is from 1:1 to 1:10.

(7) A process for asymmetric reduction of ketones with baker's yeast as defined in claim 1, wherein the aqueous solution containing the ketones is agitated in a reaction vessel, and oxygen or oxygen-containing gas is blown into the aqueous solution.

(8) A process for asymmetric reduction of ketones with baker's yeast as defined in claim 2, wherein the substrate and ethanol are divided into two-several portions and each of them is added to the aqueous solution of the reaction system each at a predetermined time interval, so that the final concentration of the substrate is from 15 to 30 g/l.

# F i g. 1

EP 0 400 239 A1

# F i g. 2

EP 0 400 239 A1

# F i g. 3

EP 0 400 239 A1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | TETRAHEDRON LETTERS, vol. 25, no. 36, 1984, pages 3979-3982, Pergamon Press Ltd, GB; K. NAKAMURA et al.: "Stereochemical control in yeast reduction" * Page 3980 * | 1 | C 12 P 7/02<br>C 12 P 7/26<br>C 12 P 7/62<br>C 12 P 11/00 |
| Y | J. CHEM. SOC., CHEM. COMMUN., 1986, pages 138-140; G. GUANTI et al.: "Baker's yeast-mediated synthesis of protected alpha-hydroxy-aldehydes" * Page 139 * | 1 | |
| Y | EP-A-0 198 440 (KANEGAFUCHI) * Claims; pages 3,7 * | 1 | |
| P,X | CHEMISTRY LETTERS, 1989, pages 1465-1466, The Chemical Society of Japan; T. KOMETANI et al.: "Baker's yeast mediated bioreduction. A new procedure using ethanol as an energy source" * Whole document * | 1 | TECHNICAL FIELDS SEARCHED (Int. Cl.5)<br><br>C 12 P |

The present search report has been drawn up for all claims

| Place of search | Date of completion of the search | Examiner |
|---|---|---|
| THE HAGUE | 14-09-1990 | DELANGHE L.L.M. |